# EUROPEAN PATENT APPLICATION

(11) **EP 0 800 832 A1**
(43) Date of publication of application: **15.10.1997**
(21) Application number: 96935416.6
(22) Date of filing: 23.10.1996
(51) Int. Cl.: A61L 9/12

(54) **PERFUME EXHALING APPARATUS**

(30) Priority: 31.10.1995 JP 306658/95; 12.01.1996 JP 21993/96
(71) Applicant: JAPAN ENERGY CORPORATION, Tokyo 105 (JP)
(72) Inventor: TAMURA, Kunimitsu, Japan Energy Corporation, Toda-shi, Saitama-ken 335 (JP); KIUCHI, Norihiro, Japan Energy Corporation, Toda-shi, Saitama-ken 335 (JP)
(74) Representative: Greenwood, John David
(86) International application number: JP9603087
(87) International publication number: WO9716211

(57) **Abstract**

The aroma diffuser 1 is composed of a main body 2 equipped with a fan means 10, and an aroma container 40 which is positioned above the fan means 10, is detachably attached to the main body 2 and contains an aroma gel in the inside thereof. The lid means 50 opens and closes an opening 40 of the aroma container40. Switch means 20 opens the lid means 50 to release the opening 40 of the aroma container 40. At the same time, the fan means is started. Thereafter, the fan means 10 is control-driven by drive control means 30 either continuously or intermittently. The aroma from the aroma container 40 is carried and diffused to the outside by means of the air flow generated by operating the fan means10.

## Description

### TECHNICAL FIELD

This invention relates to an aroma diffuser for diffusing aromatic substances in specified spaces, and in particular, to a small-sized, simple and low-cost aroma diffuser capable of intermittently diffusing aroma to deter drowsiness during vehicle operation for automobiles, ships, trains and airplanes or for work in offices. Furthermore, the aroma diffuser of this invention may be used for inducing sleep, enhancing appetite and dissipating anxiety. The aroma diffuser may be operated so as to diffuse specific aromatic substances either intermittently or continuously, and may be operated in such a manner that such diffusion stops after a predetermined time period.

### BACKGROUND ART

Conventionally, essential oils from lemon, jasmin, basil, clove, neroli, peppermint and rosemary are known as aromatic materials for awakening (or deterring sleep).

Also, human olfactory becomes fatigued when the same aroma is smelled for a long time period, resulting in a loss of the perception of the aroma. This similarly applies to the awakening aroma, so that it becomes necessary to intermittently diffuse aromas indoors.

Conventionally, many products have been devised and made available for diffusion of perfumes in vehicles, in offices or in the rooms of general households. Nonetheless, with these products, pads are either soaked in perfumes and left in the rooms or else the aroma is dispersed continuously by means of a wick placed in a perfume bottle. Thus, these products do not intermittently diffuse the aroma. As mentioned above, with these devices, the awakening effect on humans is lost as time passes, resulting in the reduction of the effect to deter drowsiness.

The present applicant proposed in Patent Application No. 6-330759 a small, simple and low-cost aroma diffuser which intermittently diffuses aromatic substances or if necessary, at controlled time intervals so as to be applicable to deter drowsiness.

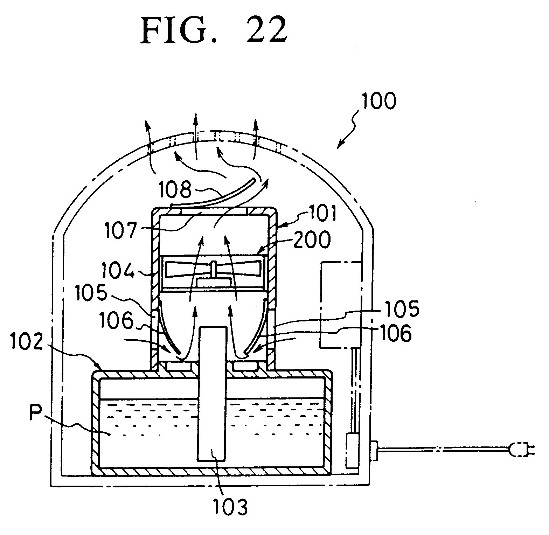

The aroma diffuser 100 is, as shown in Fig. 22, composed of a main body 101 containing a cylindrical holder 104 with a fan device 200, and an aroma container 102 which is detachably fitted into the bottom opening of the cylindrical holder 104 and has a wick 103 to suck aromatic liquid P which is stored in the interior therein. Air flow is generated only when the fan device 200 is activated. The air flows into the interior of the cylindrical holder 104 via the film-shaped gas control unit 106 which is placed at the lateral opening 105 on the cylindrical holder 104, and flows out of the cylindrical holder 104 via the gas control unit 108 on the top opening 107 of the cylindrical holder 104. By means of the air flow the aromatic substance P as sucked via the wick 104 from inside the aroma container 102, is diffused outside from the interior of the cylindrical holder 104.

The aforementioned aroma diffuser 100 operates extremely well. However, it has been found that as for the aromatic substance P which is used, gel is better suited than liquid in the light of handling.

Consequently, the present inventors experimented with the aforementioned aroma diffuser 100 having an aroma container which contains the aroma gel P in lieu of the aroma liquid P with a top opening. The result showed that in the aroma diffuser 100 aroma gel is sucked to the gas controlling units 106 and 108, and these gas controlling units 106 and 108 tend to adhere to the openings 105 and 107, necessitating a higher performance fan device 200 to ensure the opening and closing of the gas control units 106 and 108.

Additionally, the experiments by the present inventors showed that, in order to diffuse the aroma gel in the aroma container more effectively, it is necessary to induce air flow toward the opening of the aroma container and that it is crucial to generate an ascending air flow which flows around the outer periphery of the container roughly from the lower side of the aroma container towards the upperside thereof.

The present invention has been accomplished on the basis of such novel findings.

### DISCLOSURE OF THE INVENTION

An object of the invention, therefore, is to provide a small, simple and low-cost aroma diffuser with good operability, which uses an aroma gel, diffuses aroma reliably and intermittently, in which time intervals are adjustable if necessary and which is applicable as a sleep deterrent.

Another object of the present invention is to provide a small, simple and low-cost aroma diffuser with good operability, which, with the use of an aroma gel, reliably diffuses aroma, either intermittently or continuously, and is operated in such a manner that the diffusion of the aroma is stopped after the aroma has been diffused for a predetermined time, so that the aroma diffuser may be used for inducing sleep, enhancing appetite or dissipating anxiety.

The above mentioned objects are accomplished by the aroma diffuser of the present invention. In summary, this invention relates to an aroma diffuser which comprises:
a main body equipped with a fan means;
an aroma container which is positioned above said fan means and is detachably attached to said main body, and contains an aroma gel in the inside thereof;
lid means which opens and closes an opening of said aroma container;
switch means which opens said lid means to release the opening of said aroma container and is turned ON to start said fan means; and
drive control means for controlling the drive of said fan means, wherein the aroma from said aroma container is carried and diffused to the outside by means of the air flow generated by operating said fan means. Preferably, said fan means is intermittently control-driven by said drive control means after the fan means has been turned ON by said switch means . The control means can stop the drive of said fan means when a given time period has passed after said fan means was turned ON by said switch means.

According to a preferred embodiment of this invention, there is provided a cover member which is detachably attached to said main body to cover said aroma container, and said lid means is attached to said cover member. The aroma container is mounted on an aroma diffuser tray which is disposed on said main body and located above said fan means and has air vents formed at the outer periphery thereof. Thus, the aroma container can be replaced by removing said cover member.

According to another embodiment of this invention, said lid means opens the opening of said aroma container when said switch means is turned ON. Preferably, said lid means has an end held rotatably about a support axis and has an action rod rotatably attached to said end. The action rod travels either upwards or downwards, when the aforementioned switch means is turned ON. The lid means rotates about said support axis, so that the opening of said aroma container is opened, and said lid means rotates about said support axis according to the rules of gravity, whereby the opening of said aroma container is closed.

According to a preferred embodiment of this invention, there is provided switch lever means which turns ON or OFF said switch means and acts on said lid means to open or close the opening of said aroma diffuser. Preferably, the lid means has a lid cam member and said switch lever means has a cam member which is engaged with said lid cam member. The cam member pushes said lid cam member upwards by operating the operation lever formed in the switch lever means, whereby the amount of opening of said lid means with respect to said aroma container opening is adjusted.

### BRIEF DESCRIPTION OF THE DRAWINGS

Fig. 1 is a cross-sectional front view of the aroma diffuser of the first embodiment of the present invention, and shows the condition where the fan device is in operation;
Fig. 2 is a perspective view showing an embodiment of the lid device;
Fig. 3 is a side cross-sectional view of the aroma diffuser of the present invention similar to Fig. 1, and shows the condition where the fan device is in operation;
Fig. 4 is a perspective view of the aroma diffuser of the first embodiment;
Fig. 5 is a front cross-sectional view of the aroma diffuser of the present invention similar to Fig. 1, and shows the condition where the cover unit is removed in an upward direction;
Fig. 6 is a side cross-sectional view of the aroma diffuser of the second embodiment of the present invention, and shows the condition where the fan device is in operation;
Fig. 7 is a front cross-sectional view of the aroma diffuser of the present invention similar to Fig. 6, and shows the condition where the fan device is in operation;
Fig. 8 is a perspective view of the aroma diffuser of the second embodiment;
Fig. 9 is a side cross-sectional view of the aroma diffuser of the present invention similar to Fig. 6, and shows the condition where the cover unit is removed in an upward direction;
Fig. 10 is a front cross-sectional view of the aroma diffuser of the third embodiment of the present invention, and shows the condition where the fan device is not in operation;
Fig. 11 is a plan view taken along the line XI - XI in Fig. 10, and shows an embodiment of the lid device;
Fig. 12 is a front cross-sectional view of the aroma diffuser of Fig. 10, and shows the fan device in operation;
Fig. 13 is a side cross-sectional view of the aroma diffuser of the fourth embodiment of the present invention, and shows the condition where the fan device is not in operation;
Fig. 14 is a front cross-sectional view of the aroma diffuser of the present invention similar to Fig. 13, and shows the condition where the fan device is not in operation;
Fig. 15 is a perspective view of the lid device used for the aroma diffuser of Fig. 13;
Fig. 16 is a plan view showing an embodiment of the switch lever device;
Fig. 17 is a perspective view of the aroma diffuser of Fig. 13 seen from the rear;
Fig. 18 is a perspective view, seen from the front, of the aroma diffuser of Fig. 13 in the condition where the cover unit is removed in an upward direction;
Fig. 19 is a side cross-sectional view showing an aroma diffuser with a similar structure to the aroma diffuser as shown in Fig. 13, but with a difference in the structure of the switch ON/OFF recognition device;
Fig. 20 is a plan view showing an embodiment of the switch lever device of the aroma diffuser of Fig. 19;
Fig. 21 is a perspective view of the aroma diffuser of Fig. 19, seen from the front, and shows the condition where the cover unit is removed in an upward direction; and
Fig. 22 is a cross-sectional view of the aroma diffuser of a previous application, and shows the condition where the fan device is in operation.

### BEST MODE FOR CARRYING OUT THE INVENTION

Now, embodiments of the aroma diffuser according to the present invention will be described in more detail with reference to the drawings.

### Embodiment 1

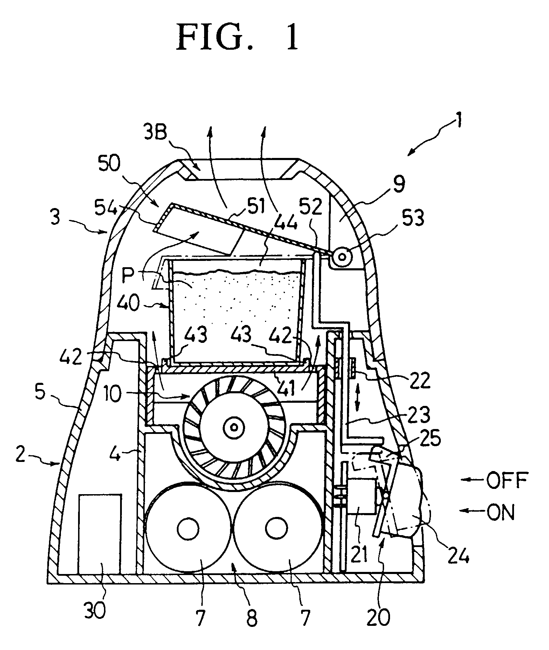

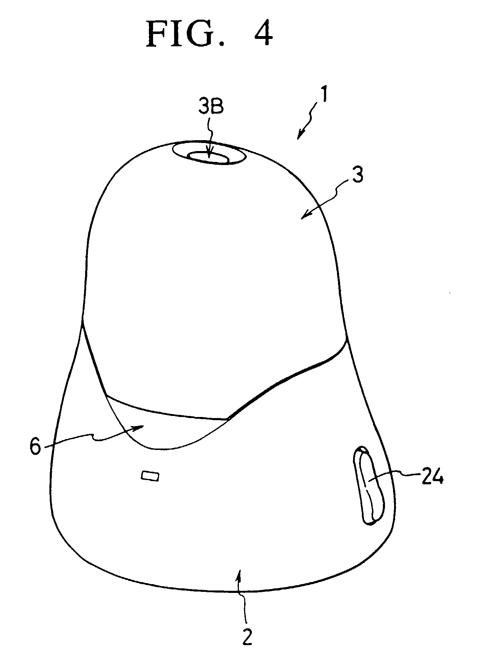

Figs. 1 to 4 show an embodiment of the aroma diffuser of the present invention. As per this invention, the aroma diffuser 1 is equipped with a main body 2 and a cover unit 3 located roughly above the main body and detachably mounted on the main body 2.

The main body 2 in this embodiment is made up of the double wall structure which comprises a roughly cylindrical holder 4 located internally and an external cover 5 encompassing this holder 4. The internal holder 4 has a battery case 8 formed below, which contains dry cell batteries 7, for example two UM-2 batteries. Disposed above the holder is a fan device 10 which generates the air flow directed upwards from the main body 2.

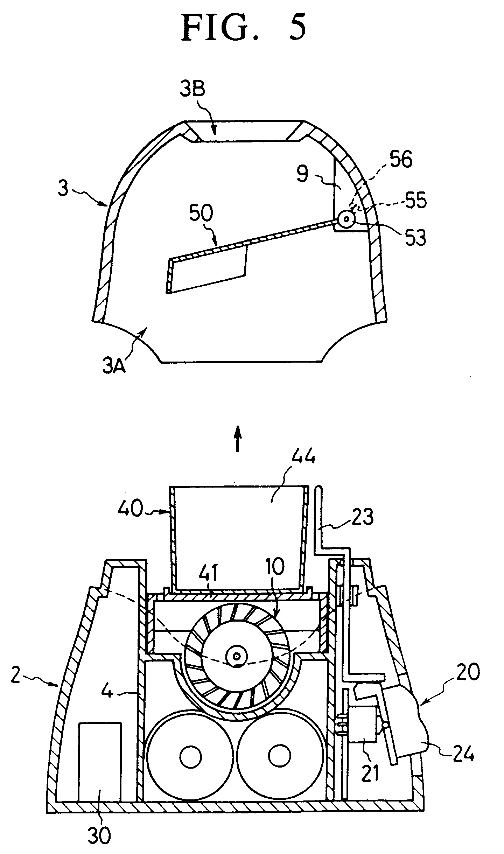

In this embodiment the aforementioned cover unit 3 is composed of a cylindrical member having an opening 3A (Fig. 5) at the lower side of the unit and a smaller opening 3B at the upper side of the unit. The opening 3A is adapted to be detachably press-fitted onto the top of the external cover unit 5 of the main body 2. The cover unit 3 may also be bolted onto the external cover 5, if desired.

As for the aforementioned fan device 10 a small-sized fan may preferably be employed, for example, which sucks air in the axial direction and expels air flow in the radial direction. The fan device 10 is powered by the dry cell battery 7 mentioned above and the ON/OFF operation and the intermittent drive control of this fan device are performed with the switch device 20 and drive control device 30 mounted on the main body 2. The switch device 20 and the drive control device 30 will be described in detail later.

The fan device 10 in this embodiment employs a mini fan directly connected to a motor such as RF-330-TK made by Mabuchi Motor Ltd, a compact motor available on the market. The performance specifications of this mini fan have a rated voltage of DC 3V, a rated current of 9mA, a rated input of 0.027W and a maximum wind capacity of 0.1 m³/m.

Also, above the internal holder 4, the tray 41 for the aroma container is installed covering the aforementioned fan device 10. At the center of the tray 41, the aroma container 40 is placed while numerous air vents 42 are formed to permit air flow upwards from the aforementioned fan device 10, encompassing the outer periphery of the aroma container 40 placed on the tray 41. Also, it is possible to provide a tab 43 in order to define the mounting position of the aroma container 40 on the upper surface of the tray 41.

The aforementioned aroma container 40 may be made of glass or plastic and in various forms. In this embodiment, the aroma container 40 is made in the form of a cup with a circular cross-section, which is made of plastic and had a thickness of 1 mm and the upper portion of which is opened. The container has an outer diameter of approximately 58 mm at the bottom and an outer diameter of 62 mm at the top opening and a height of 62 mm.

The aroma gel P contained in the aroma container 40 contains an aromatic substance, a gelatinizing agent, a dispersant, etc. As for the aromatic substance to be used, a wide range of choice is available to match the purpose of use of the aroma diffuser in the present invention. For example, in the case where the aroma diffuser of the present invention is to be used as a sleep deterrent, various materials may be used which are known as aromas for preventing sleep. For example, materials such as the essential oils of lemon, jasmin, basil, clove, neroli, peppermint, rosemary mentioned above and in addition, essential oils of eucalyptus, verbena, citronella, cajuput, salvia, thyme and hyssop; extracts of onion and garlic; formic acid; acetic acid; ethyl formate; propyl formate; acetic ester (ethyl, propyl, butyl, heptyl, nonyl, menthyl, isomenthyl, etc.), amyl nitrite, trimethyl cyclo hexanol, allyl sulphide, etc.,may be used either alone or by mixing plural kinds of said materials with each other. In the embodiment, favorable results could be obtained by use of an aromatic substance prepared by appropriately mixing lemon oil, peppermint oil, rosemary oil and L-menthol. As for the gelatinising agent, a water gel type agar product which liquefies at 75 °C may be utilized. Furthermore, additives such as colorants and bitterness enhancers may also be added.

When the aroma diffuser of the present invention is used as a sleep inducer, for the aromatic substance, nonyl alcohol, decyl alcohol, phenylethyl alcohol, methyl carbonate, ethyl carbonate, etc., in addition to essential oils of rose, jasmin, camomille, neroli, etc., which are recognized as sleep inducing aromatic substances, may be used either alone or by mixing plural kinds of the substances. Furthermore, when the aroma diffuser of the present invention is used as an appetite enhancer, carvone, estrogol, elemor, etc. in addition to essential oils such as basil, perilla, majoram, thyme, laurel, juneberbery, lemon, nutmeg, ginger, onion and garlic, which are known to enhance appetite may be used either alone or by mixing plural kinds of said substances. Futhermore, when the aroma diffuser of the present invention is used as an anxiety dissipater, citral, citronellal, borneol, linalool, geraniol, nerol, rodinol, etc., in addition to essential oils such as lavender, bergamot, lemon, majoram, rosemary, clary sage, peppermit, basil rose, jasmin, petit grain, nutmeg, cinnamon, clove, mace and ginger, which are known aromatic sunbstances for dissipating anxiety and fighting depression, may be used either alone or by mixing plural kinds of the materials with one another.

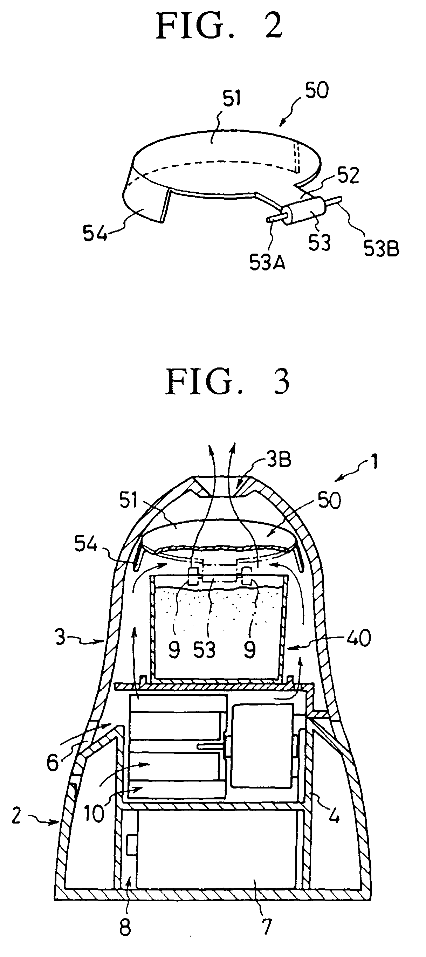

According to the present invention, additionally, a lid device 50 to open or close the top opening 44 of the aroma container 40 is installed. In this embodiment, as can be understood with reference to Fig. 2, the lid device 50 comprises a lid unit 51 in the form of a circular plate large enough to close up the top opening 44 of the aroma container 40 and a support arm 52 which is integrated with the lid unit 51. This support arm 52 is equipped, in its external end, with a rocking support rod 53. Both ends 53A and 53B of the rocking support rod 53 are rotatably held in this embodiment by the support 9 (Fig. 3) formed on the aforementioned cover unit 3. Normally, the lid device 50 is exerted to rotate counterclockwise in Fig. 1 by the weight of the lid unit 51 around the rocking support rod 53, in order to close the opening 44 of the aroma container 40.

In this embodiment, additionally, the lid device 50 is equipped, at the opposite end of the aforementioned support arm 52 of the lid unit 51, with the guide unit 54 which guides the air flow from below toward the open opening 44 of the aroma container 40, as will be described in more detail later. In this embodiment, this guide unit 54, though it can be provided on the entire periphery of the lid unit 51, is formed only on one half of the periphery. Also, it is more favorable to shape this guide unit 54 in the form of a skirt which is open more at the top than at the bottom.

In the present invention the aforementioned lid device 50 is so structured that the closing or opening movement is performed by the ON-OFF operation of the switch device 20. The switch device 20 is equipped, in this embodiment, with a seesaw switch 21 mounted on the main body 2. The main body 4, on the other hand, has the operation rod 23, which is supported by the support unit 22 formed on the main body 2 to slidably move in an up-and-down direction. The upper end of the operation rod 23 presses against the bottom surface of the support arm 52 of the aforementioned lid device 50 while the lower end abuts against the operation member 25 mounted on the push button 24 of the seesaw switch 21.

Consequently, when the switch device 20 is turned to the condition of ON, the operation member 25 of the seesaw switch 21 pushes the lower end of the operation rod 23 upward and turns, as shown in Fig. 1, the lid unit 51 clockwise. Thus, the opening 44 of the aroma container 40 is opened. When the switch device 20 is turned to OFF, the operation member 25 of the seesaw switch 21 travels to the position as indicated by the imaginary line in Fig. 1, removing the force necessary to push the lower end of the operation rod 23 upward. The lid unit 51, therefore, is rotated by gravity counterclockwise to close the opening 44 of the aroma container.

Next, the operation of the aroma diffuser 1 of this embodiment with the above mentioned structure will be described.

As is appreciated from the above, while the switch device is turned OFF, the lid device 50, that is, the lid unit 51 will be rotated counterclockwise to close the opening 44 of the aroma container 40.

Now, when the switch device 20 is turned ON, the operation member 25 of the seesaw switch 21 pushes the action rod 23 upward, as described above, to turn the lid unit 51 clockwise and to open the opening 44 of the aroma container 40, as shown in Fig. 1. At the same time, the fan device 10 is turned ON, and starts to rotate. As shown by the arrows in Figs. 1 and 3, air is introduced by the action of the fan device 10 inside the main body 2 via the air vent 6 provided between the main body 2 and the cover unit 3. This air flow passes through the vent opening 42 of the aroma container tray 41 and flows upwards along the external periphery of the aroma container 40.

According to this embodiment the air flow moving upwards inside the main body is forcibly introduced to the opening 44 of the aroma container 40 by the skirt-shaped guide unit 54 and the lid unit 51 of the lid device 50 which is raised above the aroma container 40.

The aroma gel P inside the aroma container 40 is dispersed evenly along with the evaporation of the water, carried by the air flow travelling through the opening 44 of the aforementioned aroma container and flows out of the opening 3B above the cover unit 3 to the outside.

The aromatic substance or the aroma, which flows out of the opening 3B of the cover unit into the atmosphere, is dispersed inside the vehicle, office or residential rooms in which the aroma diffuser 1 is placed.

According to the present invention the ON/OFF of the fan device 10 is effected by the switch device 20, as described above, while the control of the intermittent operation is effected by the drive control means 30. In other words, as the drive control means 30 maintains the fan device 10 at ON for a given length of time, the fan device 10 is turned OFF for a given time period to stop its operation. As the gas pressure of the aromatic substance inside the cover unit 3 reaches a certain level due to the stopping of the fan device 10, gas dispersion from the aroma container 40 is restricted or stopped, so that the outward flow from the opening 3B of the cover unit to the air outside is substantially stopped.

The intermittent drive control of the fan device 10 can be easily and inexpensively achieved by incorporating, for example, a discrete timer which is well known by those skilled in the art, in the control means 30. In mentioning one concrete example of the ON/OFF control in the case where the aroma diffuser of the present invention is used for preventing sleepiness, normally, the stop time period (OFF) and the diffusion time period (ON) are ten minutes and twenty seconds, respectively. If desired, it is also possible to change the stop time (OFF) to 20 minutes, the diffusion time (ON) to twenty seconds or the stop time (OFF) to five minutes and diffusion time (ON) to sixty seconds.

In the case where the aroma diffuser of the present invention is used for inducing sleepiness, the operation of the fan device 10 can be directed to run for a given time period, for example, two hours during which the aroma is diffused. Thereafter, when sleep has been accomplished, the operation may be directed to halt the diffusion of the aroma. In the case where the diffuser is used for enhancing appetite or for dissipating anxiety, the fan device 10 may be continuously operated and also may be controlled to run intermittently as described above.

Regarding the aroma diffuser of this embodiment, when the aromatic gel P in the aroma container 40 is completely consumed, the cover unit 3 which is fitted into the upper portion of the main body 2 is removed by applying upward force, so that the upper portion of the main body 2 can be opened. As the lid device 50 covering the opening 44 of the aroma container 40 is mounted on the cover unit 3 in this embodiment, the aroma container 40 is exposed by removing the cover unit 3 from the main body 2. Thus, the exhausted aroma container 40 on the tray 41 can easily be replaced with a new container.

Incidentally, with the structure of this embodiment the lid device 50 can be removed from the main body 2 along with the cover unit 3. However, it is also possible to have a construction that can prevent excess rotation of the lid device 50 beyond the specified position inside the cover unit 3 with the aid of a position guide piece 55 which is disposed on the periphery of the rocking support shaft, and the position guide piece 55 is in contact with the stopper 56 formed in the support portion 9 of the cover unit 3, as shown by the imaginary line in Fig. 5. With such a structure, the resetting of the cover unit 3 to the main body 2 and the closing of the opening 44 of the newly replaced aroma container 40 by the lid device is easily accomplished.

According to this embodiment, as described above, since the opening and closing of the opening 44 of the aroma container 40 can be reliably performed with the lid device 50 by operating the switch device 20, and also since the air flow through the opening 44 of the aroma diffuser 40 is generated by the fan device 10, the diffusion of the aroma is positively and as required, intermittently accomplished by using the aroma gel P. This provides an aroma diffuser which realizes miniaturization and simplification of the device and is low in cost.

When the aromatic substance is exhausted and the aroma container 40 is empty, it is possible to easily remove the exhausted aroma container 40 from the main body 2 by removing the cover unit 3, and to substitute a new aroma container 40 for the exhausted one. This brings about the advantages of making the operability extremely good.

### Embodiment 2

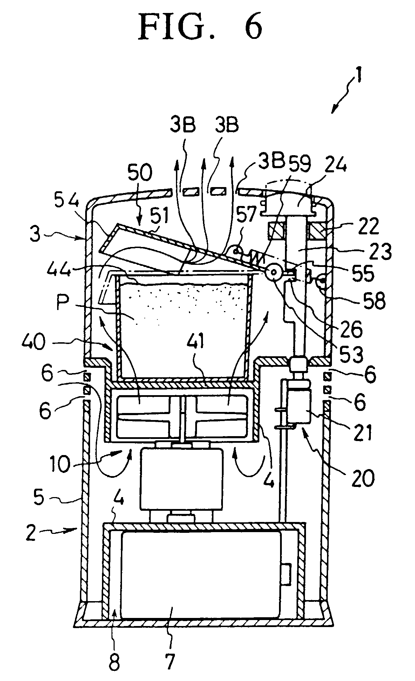

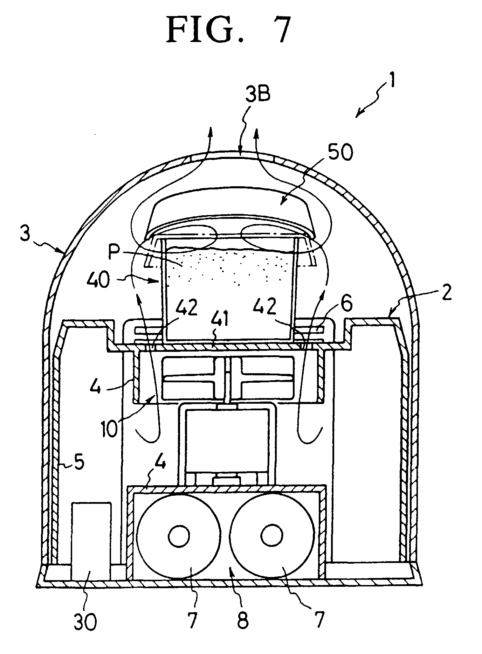

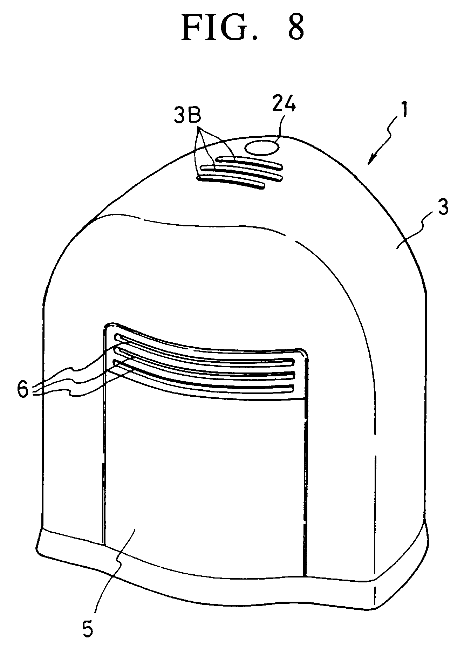

Figs. 6 to 8 show another embodiment of the aroma diffuser 1 of the present invention. The aroma diffuser 1 in this embodiment has a construction similar to the aroma diffuser 1 of Embodiment 1, so that the same reference numerals are given to the equivalent parts which have the same functions and operations as in Embodiment 1 and a detailed description is omitted.

The aroma diffuser 1 in this embodiment is equipped with a main body 2 and a cover unit 3 which is positioned roughly above the main body 2 and is detachably fixed to the main body. The main body 2 is equipped with the holder 4 in which the battery case 8 for containing, for example, two UM-2 dry cells 7 is positioned. Disposed above the holder is the fan device 10 for generating air flow upwards from the main body 2. The external cover 5 is positioned surrounding this holder 4.

The tray 41 for the aroma container is integrally formed above the internal holder by covering the aforementioned fan device 10. The aroma container 40 is mounted in the center of the support plate. The air flow from the fan device 10 is flown upwards via the vent 42 provided in the tray 41. Also, similarly to the case of Embodiment 1, bumps (not shown) to define the mounting position of the aroma container may be formed on the upper surface of the tray 41.

As for the aforementioned fan device 10, the same fan device as in Embodiment 1 may be used.

Similarly, as in Embodiment 1, in this embodiment the lid device 50 for opening and closing the upper opening 44 of the aroma container 40 is disposed. In this embodiment the switch device 20 for opening and closing the lid device 50 is the switch 21 which is a push button. Though the push button switch itself is fixed to the main body 2, the button 24 which the user directly operates is mounted on the cover unit 3. In other words, the push button 24 in this embodiment is connected to the action rod 23 extending downwards. The action rod 23 is slidably attached to the support member 22 disposed on the cover unit 3.

The lower end of the action rod 23 is in contact with the push button switch 23. Therefore, by pressing this push button 24 the action rod 23 causes the push button switch to turn ON/OFF. Also, this action rod 23 has a hollow spot 26 formed in the middle of the rod. Fitted to this hollow spot 26 is the positioning piece 55 which is formed on the periphery of the rocking support shaft 53 of the lid device 50. A tension spring 59 is placed between the hook 57 provided on the upper surface of the lid unit 51 of the lid device 50 and the hook 58 formed on the internal wall of the cover unit 3.

Therefore, in order to turn the switch device ON, when the push button 24 is pressed down to make it in the ON condition, the action rod 23 moves downwards to press the push button switch 21 and to turn it ON. At the same time, the hollow spot 26 on the action rod 23 contacts the positioning piece 23 of the lid device 50 to rotate the lid device 50 clockwise in Fig. 6 and to open the opening 44 of the aroma container 40. The lid device 50 is maintained in the opened state, even after the push button 24 is released due to the action of the aforementioned tension spring 59. Next, when the push button 24 is pressed down again to turn OFF the switch device 20, the push button switch 21 is turned OFF. At the same time the push button switch 21 pushes up the action rod 23 by the upward force of the switch 21 rotating the lid device 50 counterclockwise and closing the opening 44 of the aroma container 40. The lid device 50 is maintained in this closed state by the action of the tension spring 59.

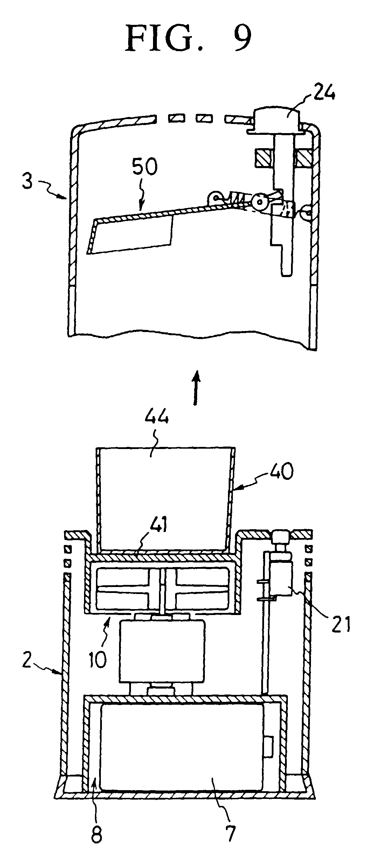

An operational description of the aroma diffuser will be omitted since the aroma diffuser 1 of Embodiment 2 operates in the same manner as the device of Embodiment 1. With the aroma diffuser 1 in this embodiment, the cover unit 3 which is fitted into the upper part of the main body, can be removed from the main body 2 by pulling the cover unit 3 upwards, as shown in Fig. 9, when the aroma gel P in the aroma container 40 is exhausted. As the lid device 50 covering the opening 44 of the aroma container is attached to the cover unit 3 in this embodiment as well, the aroma container 40 can be exposed to the outside by removing the cover unit 3 from the main body 2. Therefore, the exhausted aroma container 40 on the aroma container tray 41 can be easily replaced with a new one.

In the aforementioned Embodiments 1 and 2, the lid device 50 was described as being mechanically operated by the action rod 23 and the like which is located on the switch device 20, but this invention is not limited to this.

### Embodiment 3

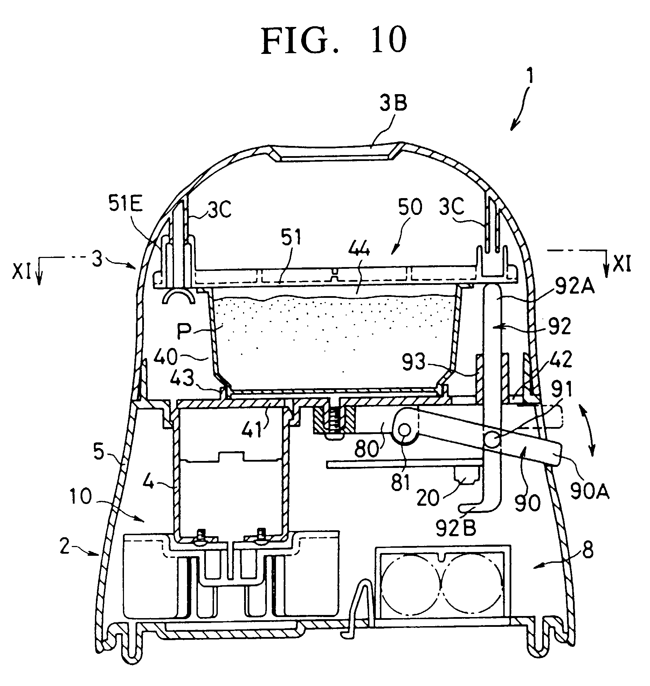

Figs. 10 to 12 illustrate still another embodiment of the aroma diffuser of the present invention.

The aroma diffuser 1 of this embodiment has a substantially similar construction to the aroma diffuser 1 of Embodiment 1. Therefore, the same reference numerals are given to the parts with the equivalent functions and operations, and a detailed description is omitted.

The aroma diffuser 1 in this embodiment comprises a main body equipped with an external cover 5 and a holder 4 made up of the internal frame structures which are integrated into an aroma container tray 41 formed integrally with the external cover 5. The cover unit 3 is disposed roughly above the main body 2 and is detachably fitted in the upper edge of the external cover 5 of the main body 2. The fan device 10 is set on the holder 4 to generate air flow upward from the main body 2. The battery case 8 for dry cell batteries, for example, two UM-2 dry cell batteries, is formed at the bottom of the holder 4.

The aroma container 40 is mounted on the center portion of the aroma container tray 41 positioned above the internal holder 4. Air flow from the aforementioned fan device 10 is sent upwards through the vents 42 formed suitably for the purpose on the tray 41. Also, similarly, as in Embodiment 1, the bump 43 to define the mounting position of the aroma container 40 is formed on the tray 41.

The fan device 10 in this embodiment employs a mini fan directly connected to a compact motor which is available on the market, such as the RF-330-TK-07800 made by Mabuchi Motor Ltd. The performance specifications of this mini fan include a rated voltage of DC 3V, a rated current of 9mA, a rated input of 0.027W and a maximum wind capacity of 0.1 m3/minute. Naturally, a fan device similar to that in Embodiment 1 may be employed.

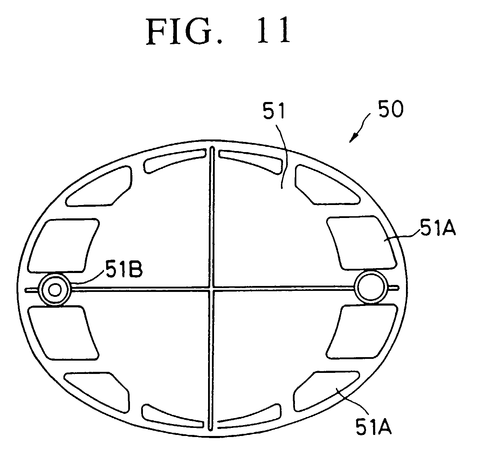

In this embodiment, similarly, the lid device 50 for opening and closing the upper opening 44 of the aroma container 40 is disposed. However, in this embodiment, the lid unit 51, the center portion of which covers the upper opening 44 of the aroma container 40 and at the outer periphery of which, as shown in Fig. 11, the openings 51A are formed, is swingably attached to a support axis 3C by a cylindrical hook device 51E formed on one of its ends. The support axis 3C hangs vertically downward from the top of the cover unit 3.

On the other hand, below the aforementioned aroma container tray 41, the rocking lever 90 is swingably supported by the tray plate 80 integrally formed with the tray 41 through the rotation axis 81. One end 90A of this rocking lever 90 (the right side end in Fig. 10) extends to the outside from the external cover 5 to function as a switch lever. Also, the action rod 92 is rotatably connected to this switch lever 90 through the connecting axis 91. This action rod 92 extends upwards through the inside of the guide cylinder 93 formed in the aroma container tray 41 and the tip 92A of the rod 92 abuts against the bottom surface of the opposite end of the lid unit 51 to the cylindrical hook device 51E. Also, the lower end 92B of this action rod 92 is bent at right angles to thereby form the operational part of a switch device 20 such as a micro switch. The switch device 20 is attached to the tray 41 by means of a support unit (not shown).

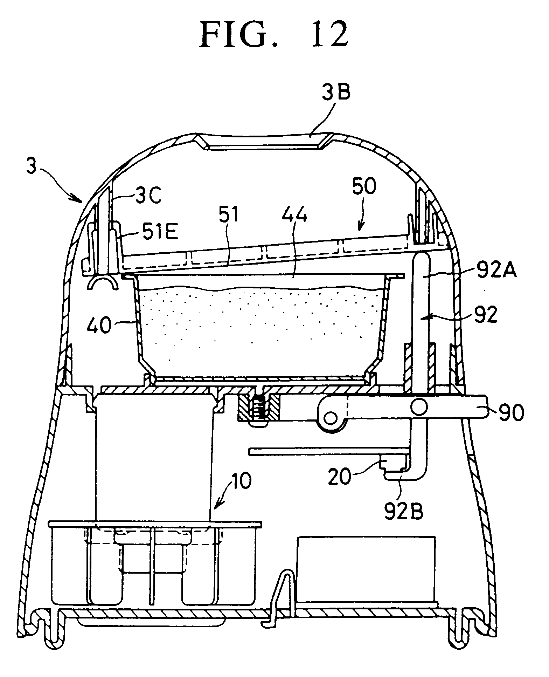

Therefore, when the switch lever 90 is lifted upwards, as shown in Fig. 12, the action rod 92 also travels upwards and its tip 92A lifts the lid unit 51 of the lid device 50. The lid unit 51 rotates in the counterclockwise direction in Fig. 12, making the cylindrical hook device 51E the center of rocking movement, so that the lid device 50 opens the opening 44 of the aroma container 40. At the same time, the switch device 20 is turned ON by the action part 92B of the lower end of the action rod 92. As a result, the fan device 10 is exerted, so that the aroma which has been dispersed from the opened aroma container 40 is entrained by the air flow flowing through the opening 3B above the cover unit 3 and is dispursed out of the opening 44 of the aroma container 40 to the outside. On the other hand, if the switch lever 90 is pressed down, the action rod 92 will also travel downward and, as shown in Fig. 10, the lid device 50 will close the opening 44 of the aroma container 40. At the same time, as for the action rod 92, its operating part 92B leaves the switch device 20, thereby turning OFF the switch device 20 to stop the fan device 10.

An operational description is omitted since the aroma diffuser 1 in this Embodiment 3 can be operated similarly as in the apparatus of the previous embodiments. In the aroma diffuser 1 of the present embodiment, the cover unit 3 fitted in the upper portion of the main body 2 can be removed from the main body 2 by pulling the cover unit 3 upwards. As the lid device 50 which covers the opening 44 of the aroma container is mounted on the cover unit 3 also in this embodiment, the aroma container 40 can be exposed by removing the cover unit 3 from the main body 2. Consequently, when the aroma gel P inside the aroma container 40 is exhausted, the emptied aroma container on the aroma container tray 41 can easily be replaced with a new one.

### Embodiment 4

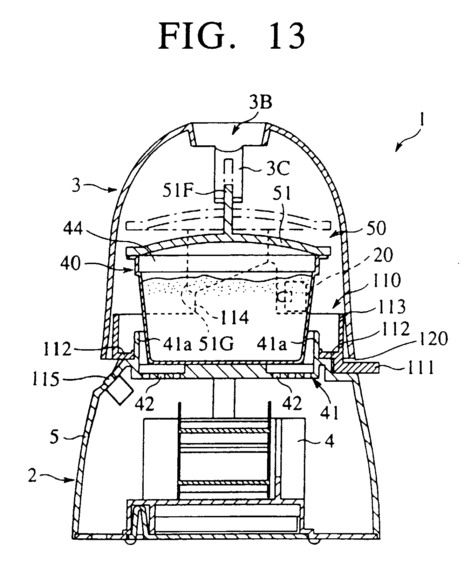

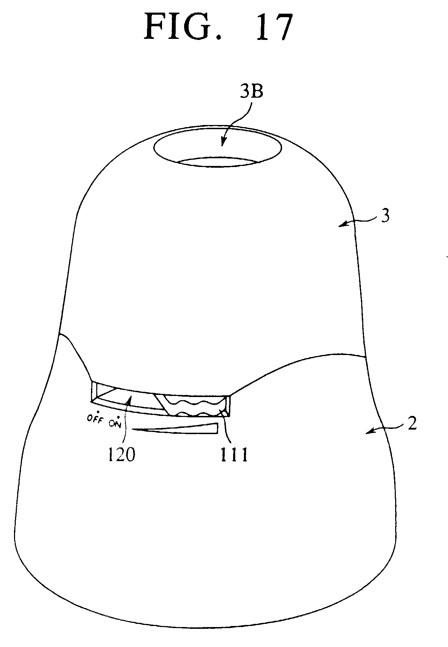

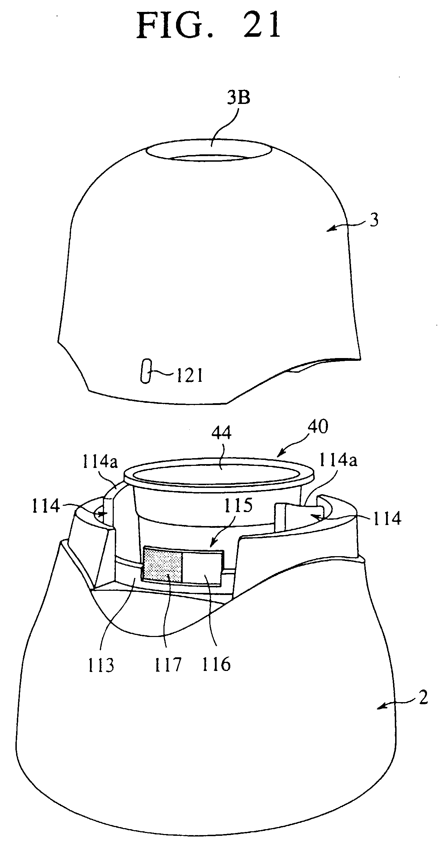

Another embodiment of the aroma diffuser 1 of the present invention is shown in Figs. 13 to 21. The aroma diffuser of this embodiment is structured similarly as in the aroma diffuser 1 in Embodiment 1. The same reference numerals are given to the parts with the equivalent functions and operations, and a detailed description is omitted.

The aroma diffuser 1 of this embodiment is composed of a main body 2 and a cover unit 3 which is roughly situated above the main body 2 and is detachably attached to the main body 2. The main body 2 is equipped with a holder 4. The holder 4 has a battery case 8 formed to accommodate, for example, UM-2 dry cell battery. Disposed above it is the fan device 10 for generating air flow upwards from the main body 2. The external cover 5 is positioned to cover this holder 4.

The tray 41 for the aroma container is installed in consolidation, covering the aforementioned fan device 10 above this internal holder 4. The aroma container 40 is mounted at the center of the tray. Air flow from the aforementioned fan device 10 is guided upwards via the vents 42 provided on the tray 41. The position and size of the vents 42 is determined to permit smooth dispersion of the aroma through the opening 3B after remaining in the top of the aroma container 40. For instance, the vents 42 are placed, partially along the periphery of the aroma container 40. On the upper surface of the tray 41, as in the case of Embodiment 1, bumps may be provided (not shown) to restrict the mounting position of the aroma container 40.

A similar fan device as in Embodiment 1 may be used for the aforementioned fan device 10.

A lid device 50 is also provided in this embodiment, as in Embodiment 1, for opening and closing the top opening 44 of the aroma container 40. In this embodiment, operation of the switch lever device 110 and operation lever 111 which are installed on the main body 2 realizes opening/closing of the lid device 50 and turns ON/OFF the switch device.

More particularly, as is understood with reference to Figs. 13 to 16, the lid device 50 is equipped with the lid unit 51, which is concave at its approximate middle, covering the top opening 44 of the aroma container 40. On the external periphery of the lid unit 51, the lid cam unit 51G which extends downwards, is provided, ideally in two diagonally positioned pairs in a diametrical direction. The action of this lid cam unit 51G will be described later in the text. By shaping the lid unit 51 in a rough convex, high in the middle, air flow via the air vents 42 is forced to remain at the top of the aroma container 40 for a short period of time, making the dispersion of aroma effective. Also, this lid unit 51 can be attached, with free sliding movement, to the support axis 3C which hangs vertically from the area of the opening 3B above the cover unit 3 due to the support hook device 51F, which is provided in the center area of the lid unit 51 and which extends upwards. The lid unit 51, therefore, is mounted under normal circumstances in such a way that it hangs by its weight above the aroma container 40, as shown in Figs. 23 and 24, closing the opening 44 of the container.

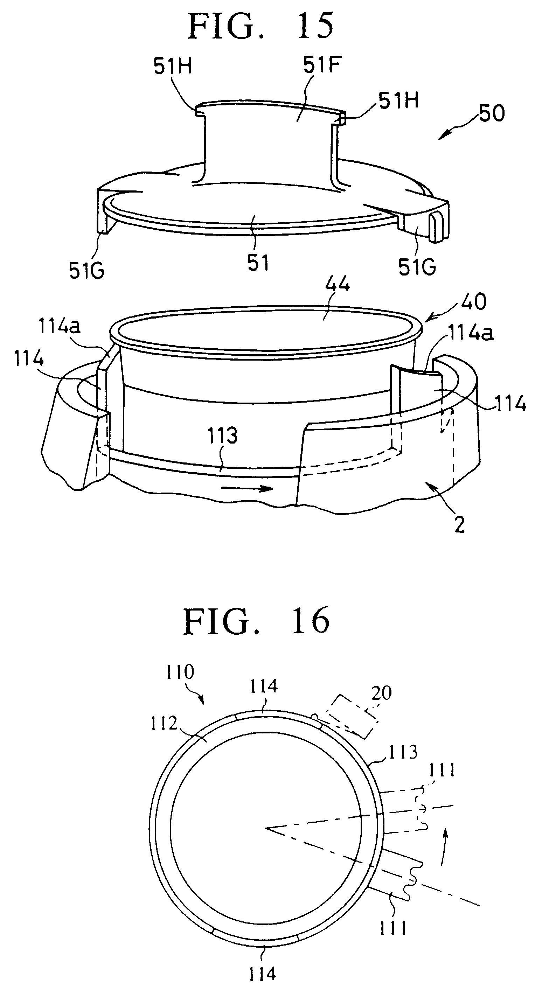

At the same time, the ring-shaped support part 112 of the switch lever device 110 fits, with free rotating movement, the ring-shaped guide 41a provided on the outer periphery of the aroma container tray 41. The outer periphery of the ring-shaped support part 112 is made in the form of a sleeve equipped with as outer wall 113 which extends upwards. An operation lever 111, which projects from the outer wall 113, passing through the narrow opening 120 provided in the border area between the main body 120 and the cover unit 3 is consolidated to the outer wall 113. Also, a cam unit 114, which extends further upwards, is provided on part of the aforementioned outer wall 113. Preferably, as is understood with reference to Figs. 11 and 16, the cam unit 114 is arranged in two pairs in diagonally opposed positions in dimetrical direction. The top edge 114a of the cam unit 114 is slanted, The top edge 114a of the cam unit 114 is structured to contact in mutually complementary relation with the shape of the bottom edge of the lid cam unit 51G provided on the outer periphery of the aforementioned lid unit 51.

Consequently, by operating the aforementioned operation lever 111 and moving this operation lever in the direction of the arrow in Figs. 15 and 16, the cam unit 114 of the switch lever device 110 rotates counterclockwise as per Figs. 15 and 16, and causes it to push upwards the lid cam unit 51G of the lid unit 51 which contacts the cam unit 114. In other words, the shift upward of the cam unit 51G lifts the lid unit 51 upwards by virtue of its support hook device 51F travelling upwards along the support axis 3C, so that the opening 44 of the aroma container 40 is opened. Also, the level of operation of the operation lever 111 or the position at which the operation ends (angle of operational shift) determines the level of the upward travel by the lid cam unit 51G and lid cam unit 51, therby adjusting the level of the opening 44 of the aroma container, namely, the level of aroma diffusion.

Additionally, a switch device 20 which resembles a micro-switch is installed on the outer periphery of the aroma container tray 41. The device 20 under normal circumstances, as shown in Figs. 13 and 16, is kept in the OFF position by the cam unit 114. And by operating the operation lever 111, as described above, the cam unit 114 rotates and the lid device 31 is lifted upwards, which turns the switch device to the ON state. Namely, the switch lever device 110 has the function of lifting the lid device 51 as well as the function of operating the switch lever.

When the switch device 20 is turned ON, the fan device 10 is driven, so that the aroma, which has been dispersed from the now opened aroma container 40, is carried by the air flow which runs through the opening 44 of the aroma container 40, and flows out into the atmosphere via the opening 3B above the cover unit 3.

On the contrary, when the operation lever 111 is operated in the opposite direction from the above description, the force of the cam unit 114 to lift the lid unit 51G upward is lost, so that the cam unit 51G and the lid unit 51 travel downwards, causing the lid unit 51 to close the opening 44 of the aroma container 40. At the same time, the cam unit 114 works on the switch device 20, bringing it to the OFF position, which in turn stops the fan device 10.

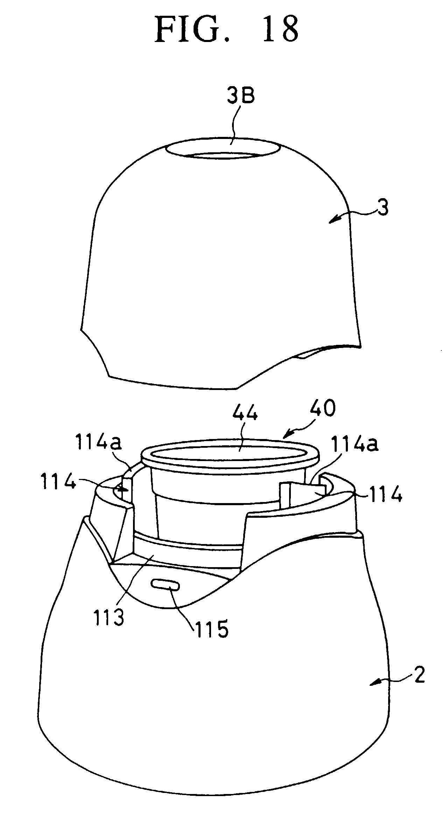

As can be seen in Figs. 13 and 18, the switch ON/OFF discerning device 115 like LED is installed in the frontal area of the main body 2. The electric switch ON/OFF discerning device 115 is structured to light up or extinguish in conjunction with the ON/OFF condition of the switch device 20.

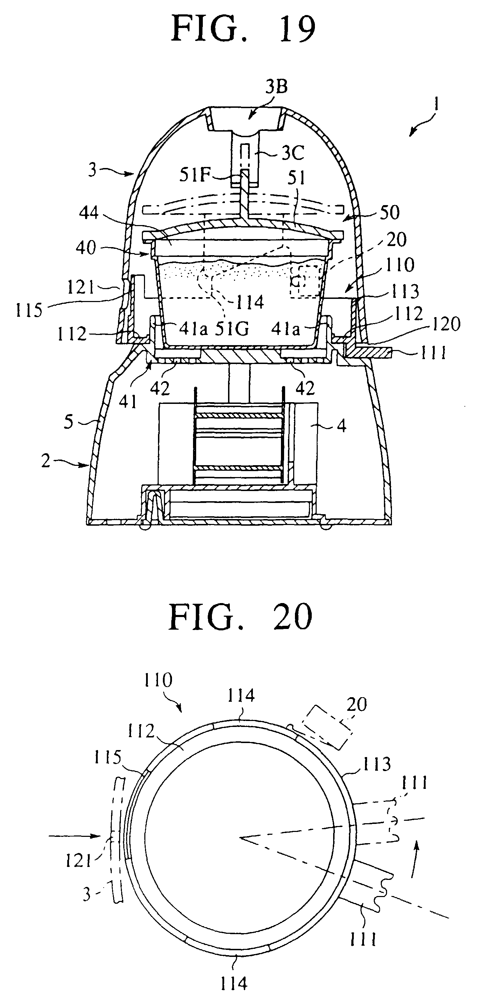

As can be seen clearly in Figs. 19, 20 and 21, the switch ON/OFF discerning unit 115 can be integrated into the outer periphery wall of the switch lever device 110 instead of the aforementioned LED 115. To wit, this discerning device 115 can be designed with a color distinction such as a red color 116 and a blue color 117 for the outer perimeter wall 113. When the switch device 20 is turned OFF, the red color 116 can be seen through the observation aperture 121 provided on the cover unit 3. When, however, the operation lever 111 is moved and the switch device 20 is turned ON, this discerning device 115 also is turned along with the operation lever 111, while the blue color 117 can be observed through the observation aperture 121. This switch type ON/OFF discerning device 115 is more advantageous in terms of power consumption than such ON/OFF methods of the electric switch ON/OFF discerning device 115 used in conjunction with the switch device 20.

Operational description is omitted since this aroma diffuser 1 of Embodiment 4 is controlled in a similar way to the equipment in the previous embodiments and can be operated intermittently or continuously.

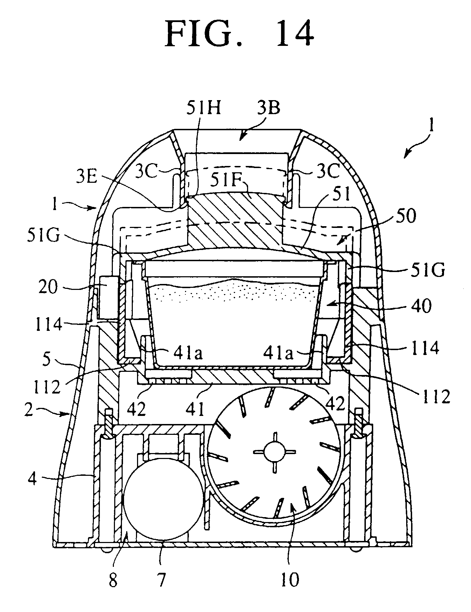

Also regarding the aroma diffuser of the present embodiment, the cover unit 3 can be removed from the main body 2 by pulling upwards the cover unit 2 fitted onto the top of the main body 2. At this moment, the lid device 50 covering the opening 44 of the aroma container remains attached to the cover unit 3, as shown in Fig. 14, as the movement of the tab 51H which is installed at the tip of the support hook device 51F is hampered, preventing any further downward movement of the lid unit 51. Thus, as in this embodiment as well, the lid device 50 is still attached to the cover unit 3, even when the cover unit 3 is removed from the main body 2, resulting in the exposure of the aroma container 40. Consequently, when the aroma gel P in the aroma container 40 has been exhausted, the finished aroma container 40 on the aroma container tray 41 can be easily replaced with a new one.

### INDUSTRIAL APPLICABILITY

As described above, the aroma diffuser of the present invention comprises a main body equipped with a fan means; an aroma container which is positioned above said fan means and is detachably attached to said main body, and contains an aroma gel in the inside thereof; lid means which opens and closes an opening of said aroma container; switch means which opens said lid means to release an opening of said aroma container and is turned ON to start said fan means; and drive control means for controlling the drive of said fan means, wherein the aroma from said aroma container is carried and diffused to the outside by means of the air flow generated by operating said fan means. Therefore, the aroma gel is used for reliable and intermittent dispersion, and, where necessary, the time interval can be controlled to permit the use as a sleep inhibitor. Furthermore, the design is compact, simple and low-priced in addition to having good operability with extremely high effectiveness. Additionally, the aroma diffuser of the present invention not only diffuses aromatic substance reliably but also can be operated to diffuse either intermittently or continuously. Furthermore, the present aroma diffuser can be operated to stop after diffusion for a specified length of time, permitting the use as a sleep inducer, appetite enhancer or anxiety dissipater.

## Claims

1. An aroma diffuser comprising:
a main body equipped with a fan means;
an aroma container which is positioned above said fan means and is detachably attached to said main body, and contains an aroma gel in the inside thereof;
lid means which opens and closes an opening of said aroma container;
switch means which opens said lid means to release the opening of said aroma container and is turned ON to start said fan means; and
drive control means for controlling the drive of said fan means, wherein the aroma from said aroma container is carried and diffused to the outside by means of the air flow generated by operating said fan means.

2. The aroma diffuser according to claim 1, wherein said fan means is intermittently control-driven by said drive control means after the fan means has been turned ON by said switch means .

3. The aroma diffuser according to claim 1 or 3, wherein said control means can stop the drive of said fan means when a given time period has passed after said fan means has been turned ON by said switch means.

4. The aroma diffuser according to claim 1, 2 or 3, further comprising a cover member which is detachably attached to said main body to cover said aroma container, said lid means being attached to said cover member.

5. The aroma diffuser according to claim 4, wherein said aroma container is mounted on an aroma diffuser tray which is disposed on said main body and located above said fan means, and has air vents formed at the outer periphery thereof, and said aroma container can be replaced by removing said cover member.

6. The aroma diffuser according to any one of claims 1 to 5, wherein said lid means opens the opening of said aroma container when said switch means is turned ON.

7. The aroma diffuser according to claim 6, wherein said lid means has an end held rotatably about a support axis and has an action rod rotatably attached to said end, said action rod travels either upwards or downwards, when the aforementioned switch means is turned ON, and said lid means rotates about said support axis, so that the opening of said aroma container is opened, and said lid means rotates about said support axis by gravity. whereby the opening of said aroma container is closed.

8. The aroma diffuser according to any one of claims 1 to 5, further comprising switch lever means which turns ON or OFF said switch means and acts on said lid means to open or close the opening of said aroma diffuser.

9. The aroma diffuser according to claim 8, wherein said lid means has a lid cam member and said switch lever means has a cam member which is engaged with said lid cam member, and said cam member pushes said lid cam member upwards by operating the operation lever formed in the switch lever means, whereby the amount of opening of said lid means with respect to said aroma container opening is adjusted.
